# EUROPEAN PATENT SPECIFICATION

(11) **EP 1 617 202 B1**
(45) Date of publication and mention of the grant of the patent: **23.09.2009**
(21) Application number: 04291832.6
(22) Date of filing: 13.07.2004
(51) Int. Cl.: G01N 21/43, G01N 21/05, G01N 21/85, G01F 1/74

(54) **Detector for distinguishing phases in a multiphase fluid mixture**
Detektor zum Unterscheiden von Phasen in einer Multi-Phasen Flüssigkeitsmischung
Détecteur pour la discrimination des phases dans un mélange fluide multiphase

(43) Date of publication of application: 18.01.2006
(73) Proprietor: SERVICES PETROLIERS SCHLUMBERGER, 75007 Paris (FR); SCHLUMBERGER TECHNOLOGY B.V., 2514 JG Den Haag (NL); SCHLUMBERGER HOLDINGS LIMITED, Road Town, Tortola (VG)
(72) Inventor: Guieze, Paul, Glatigny, 77370 Fontenailles (FR); Pinguet, Bruno, Bergen 5072 (NO)
(74) Representative: Vandermolen, Mathieu

(56) References cited:
- EP-A- 0 333 939
- US-A- 3 898 637
- US-A- 4 210 809
- US-A- 4 659 218
- US-A- 5 046 854
- US-A- 5 455 423
- US-A- 5 466 946
- US-A- 5 831 743
- US-A- 5 956 132
- WOLFFENBUTTEL B.M.A. ET AL: 'NOVEL METHOD FOR NON-INTRUSIVE MEASUREMENT OF VELOCITY AND SLUG LENGTH IN TWO- AND THREE-PHASE SLUG FLOW IN CAPILLARIES' MEASUREMENT SCIENCE AND TECHNOLOGY vol. 13, no. 10, 2002, BRISTOL, GB, pages 1540 - 1544, XP001201598
- HAMAD F.A. ET AL: 'An optical probe for measurements in liquid - liquid two-phase flow' MEASUREMENT SCIENCE AND TECHNOLOGY vol. 8, no. 10, October 1997, BRISTOL, GB, pages 1122 - 1132, XP000752147
- FORDHAM E.J. ET AL: 'Multi-phase-fluid discrimination with local fibre-optical probes: II. Gas/liquid flows' MEASUREMENT SCIENCE AND TECHNOLOGY vol. 10, no. 12, December 1999, BRISTOL, GB, pages 1338 - 1346, XP000935558
- FORDHAM E.J. ET AL: 'Multi-phase-fluid discrimination with local fibre-optical probes: III. Three-phase flows' MEASUREMENT SCIENCE AND TECHNOLOGY vol. 10, no. 12, December 1999, BRISTOL, GB, pages 1347 - 1352, XP000935559

## Description

### FIELD OF THE INVENTION

The invention relates to a detector for distinguishing a phase from a multiphase fluid mixture. The detector comprises an optical probe for optically distinguishing one of the phases from the multiphase fluid mixture based on the refractive index of the phases.
A particular application of the invention relates to the detection of the various phases of a multiphase fluid mixture from a hydrocarbon well. Such a multiphase fluid mixture typically comprises three phases: an aqueous phase, a liquid hydrocarbon phase and a gaseous hydrocarbon phase.
The invention also relates to particular applications and arrangements of the detector to measurement in the oil industry.

### BACKGROUND OF THE INVENTION

After a hydrocarbon well has been drilled and made safe, a well testing operation is generally carried out for a short period of time. The well testing operation serves to characterize the various components of the effluent flowing out of well, and to estimate the production capacities of the well. Once the well testing operation is finished, a well producing operation is carried out as long as the oil produced is satisfactory in term of quality, flowing rate, etc...
At the beginning of the well testing operation, the composition of the effluent varies considerably. The well is initially filled with aqueous residues from the well construction operation such as drilling fluid and completion fluid. The effluent that is initially collected is essentially made up of water. Subsequently, the percentage of aqueous residue decreases gradually, and the composition of the effluent becomes enriched with oil and with gas. Thus, the effluent is a multiphase fluid mixture.
During the testing and producing operation, an important concern is to identify as accurately as possible the various phases that constitute the multiphase fluid mixture.

For distinguishing a phase from a multiphase fluid mixture, it is known to perform visual observation of the multiphase fluid mixture flowing through a well flowing line. A visual observation can be performed by way of a universal fluid phase detector.

The universal fluid phase detector mainly comprises sight-glass disposed on the well flowing line. A visual observation can be performed through the glass directly by an operator or recorded by a video camera for subsequent interpretation. The phase sensing can also be performed using an optical probe based on light attenuation. These visual observations induce complex mechanical system and safety problems when operating at high pressure and high temperature. Moreover, the visual observations are by nature limited by dirty windows, wettability issues and inaccuracy or misinterpretation from operators.

Alternatively, specific fluid detectors are known for sensing a particular component of a phase using a specific physical property (conductivity, specific light absorption, etc...). These detectors are limited to one phase amongst the others.

US 5 046 854 describes a photometric probe having separate windows and a metallic probe body or cell body. The window(s) are sealed to the probe or cell body by brazed or frit seals which are less likely to leak after extended use in high temperature and/or high pressure industrial applications than probes and cells having gasket sealed windows.

EP 0 333 939 describes universal flow cells for simultaneously and/or separately performing of all kinds of single or multiple light beam measurements. The detector flow cells are characterized by a capillary flow channel passing transversely through one or more optical fibres and/or several optical fibres extending transversely from the flow channel.

US 5,956,132 describes an apparatus comprising an optical probe for discriminating between the three phases of a moving fluid comprising gas, oil and water.
The optical probe comprises a detector block having a sensitive zone in contact with the moving fluid. An incident light beam injected in the detector block enables to discriminate between the three phases by using a measurement technique based on the reflection of light induced by the specific refractive indices of each phase.
The apparatus, in particular the detector block is placed in the moving three-phase fluid flowing along the oil well line. The oil well line has an internal diameter of a few centimeters. Because of the oil well line dimension, a plurality of detectors is placed at different distances from the axis of the well and at different azimuths, so as to obtain a three-dimensional image of the phases in the flowing fluid. Besides, because of the internal diameter of the oil well line and the position of the optical probe in the line, wettability issues and dirt deposit on the probe are a source of inaccuracy.

### SUMMARY OF THE INVENTION

One goal of the invention is to propose a detector for distinguishing a phase from a multiphase fluid mixture based on optical measurement that has a better accuracy than the prior art measurement apparatus and system.

According to the invention, a detector comprising an optical probe coupled to a detector cell is designed in such a way that each phase of the multiphase fluid mixture circulates around a probe tip.
More precisely, the present invention relates to a detector for distinguishing a phase from a multiphase fluid mixture, the detector comprising:
- an optical probe for optically distinguishing one of the phases from the multiphase fluid mixture, the optical probe having a tip in contact with the multiphase fluid mixture, and
- a detector cell arranged to be coupled to a multiphase fluid mixture flowing line and to the optical probe.
The detector cell further comprises:
- an internal flowing line arranged so that the phases of the multiphase fluid mixture flowing in the internal flowing line are driven-on by capillarity effect,
- a bore coupled to the internal flowing line for positioning the optical probe in the detector cell so that the multiphase fluid mixture flows substantially around the tip of the optical probe.

The internal flowing line is dimensioned so that the phases of the multiphase fluid mixture flowing in the internal flowing line are driven-on by capillarity effect. Due to the capillarity effect, the fluid mixture will either flow as plugs and rings or as a biphasic emulsion (in case the fluid mixture is an emulsion of water and oil, of oil and gas, etc...). The internal flowing line has an internal diameter substantially corresponding to a capillary tube diameter. For example, the capillary tube diameter may range from 0,5 mm to 20 mm. However, this range typically varies depending on the fluid mixture properties.

The optical probe is coupled to the bore of the detector cell through a compression fitting and connected to an electronic module though an optical fiber.

Consequently, with the detector according to the invention, the multiphase fluid mixture is driven-on by capillarity effects and is forced to circulate around the optical probe (typically at a flowrate around a few cm/s, e.g. 3 cm/s to 15 cm/s). These characteristics enable an auto-cleaning of the optical probe tip limiting any dirt deposit on the optical probe and improving accuracy of the whole detector.

The detector according to the invention is compact, light, safe and accurate. The detector constitutes a compact hand-held equipment that can be used in full autonomy or connected to an acquisition system. The detector can withstand high-pressure (higher than 700 bar) and high temperatures (higher than 150 °C) that are expected in oil and gas field surface, down-hole and subsea operations. The detector is advantageously compliant with the safety regulation for being operated in hazardous area.

In a first application, the detector according to the invention is used as an in-line phase detector allowing sensing gas, oil/condensate and water during sampling or bottle transfer down-hole or in sub-sea operations. In such an application, the detector according to the invention enables to ensure that the sampled phase is the expected one, and enables to sense the interface between the various phases during fluid transfer operations from vessel to vessel (a vessel being a generic terminology for e.g. sampler, shipping cylinder, PVT cell, pycnometer, flash apparatus, etc...).
In this application, the detector is integrated in an arrangement for transferring a single phase from a sampling bottle comprising a multiphase fluid mixture to a shipping bottle. The transferring arrangement comprises a flow line coupling the sampling bottle to the shipping bottle, a pumping arrangement for transferring a fluid from the sampling bottle to the shipping bottle, and a detector according to the invention.

In a second application, the detector according to the invention is used as an in-line phase ratios monitoring application. In such an application, the detector according to the invention enables to accurately measure the fractions of each phase in the multiphase fluid mixture. This is also advantageous for improving measurement made by other oil-water-gas fraction measuring apparatus. In this case, the detector according to the invention is used as a redundancy device or as a recalibration device.
In this application, the detector is integrated in an arrangement for testing a well that produces a multiphase fluid mixture flowing through a main line. The arrangement comprises a derivation line coupled to the main line for deriving a part of the multiphase fluid mixture, a detector according to the invention and an acquisition device connected to the detector.

In a third application, the detector according to the invention is used as a flow rate measuring apparatus. The arrangement for measuring a flowrate of a phase of a multiphase fluid mixture flowing though a main line of a hydrocarbon well comprises a derivation line coupled to the main line for deriving a part of the multiphase fluid mixture, at least two detectors according to the invention, each detector being connected in serial along the derivation line and an acquisition device connected to the detectors. The acquisition device receives from each detector a signal which level is characteristic of the phase flowing around the tip, performs a cross correlation of the received signals, determines a transit time of each phase from one detector to the other, and computes a measurement representative of the flow rate in the main line.

### BRIEF DESCRIPTION OF THE DRAWINGS

The present invention is illustrated by way of example and not limited to the accompanying figures, in which like references indicate similar elements:
Figure 1 schematically illustrates a detector for distinguishing a phase from a multiphase fluid mixture according to the invention;
Figures 2.A, 2.B and 2.C schematically illustrate various embodiments of the detector cell of the invention;
Figure 3 schematically illustrates the preferred embodiments of the detector cell of the invention comprising an optical probe;
Figure 4 schematically illustrates the known operation principle of the optical probe;
Figure 5 schematically illustrates a first application of the invention, namely a stand-alone phase interface sensing application;
Figure 6 schematically illustrates a second application of the invention, namely an in-line phase ratios monitoring application;
Figure 7 shows a typical signal over time graphic obtained in the second application of the invention shown in Figure 6.

### DETAILED DESCRIPTION OF THE INVENTION

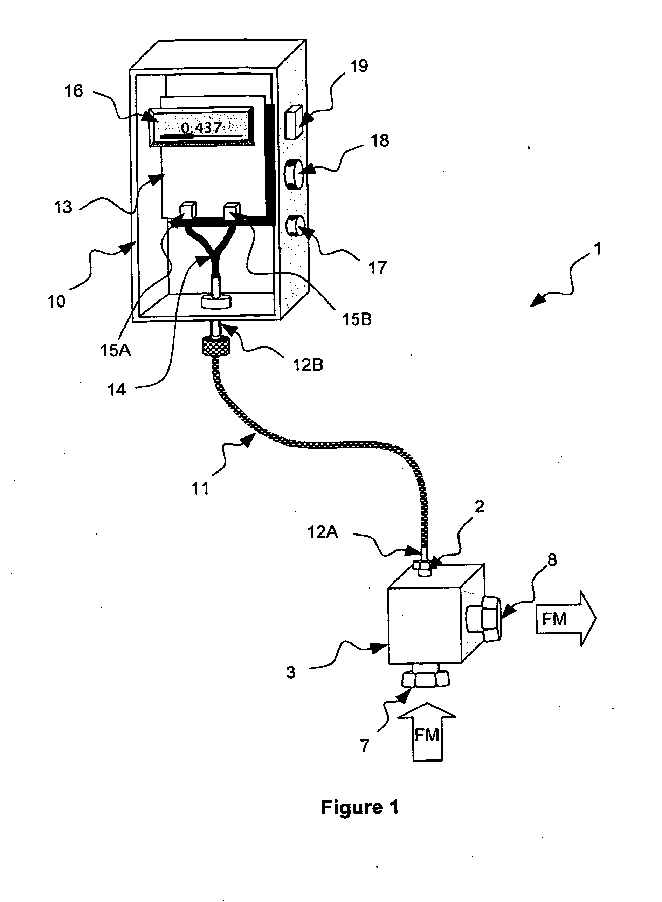

Figure 1 shows a detector 1 for distinguishing a phase from a multiphase fluid mixture. The detector comprises a detector cell 3 connected to an electronic module 10.

The detector cell 3 comprises an input connection 7 and an output connection 8 that are connectable to a flow line (not shown) in which the multiphase fluid mixture FM flows. Preferably, the input 7 and output connections 8 are high-pressure connection. The detector cell 3 further comprises an optical probe 2 coupled to an optical fiber 11 by an optical connector 12A. The optical fiber 11 connects the optical probe 2 to the electronic module 10. Advantageously, the optical fiber 11 is an armored optical fiber.

The detector cell will be described in greater details here below.

The electronic module 10 has the form of a hand-held housing. The electronic module 10 comprises an optical connector 12B for optically connecting the module to the optical fiber 11. The electronic module 10 further comprises a circuit board 13. The circuit board 13 comprises electronic elements, in particular an emitting diode 15A and detecting diode 15B, and other electronic elements (not shown) for ensuring the functionality of the detector.
The emitting and detecting diodes are connected to the optical fiber 11 via an optical coupling 14. The optical coupling 14 is an optical fiber assembly having a Y shape that connects the optical probe to both emitting and detecting diodes.
The emitting diode is advantageously a laser type diode. The detecting diode is advantageously a photo-transistor.
The electronic module 10 also comprises a display 16, a power switch 19, and a power supply connection 17. The electronic module 10 may also comprise an input/output connector 18. All these elements are connected to the circuit board 13. The circuit board 13 controls the light emission by the emitting diode 15A, the light detection by the detecting diode 15B, the display and eventually the signal input/output via the input/output connector 18.
The display 16 can comprise 3 LEDs, each LEDs indicating which phase is currently in contact with the optical probe. The display 16 can also be of a more sophisticated type, e.g. LCD display, giving further information about the detector status, information related to measurement performed and/or graphical evolution of measurements versus time.
Advantageously, the electronic module 10 comprises an internal power supply under the form of a battery (not shown) which enables a full autonomous mode. Advantageously, in safe area operation, the alimentation connection 17 can be used as a direct power supply or as a battery charging input. Advantageously, the input/output connector 18 can be designed for connection to various peripheral (e.g. computer, printer, etc...). In particular, the input/output connector 18 can deliver an output signal (for example a 4 mA to 20 mA signal) for allowing the acquisition of the detector response versus time on a computer, or any other acquisition devices.
The electronic module is advantageously designed and built according to safety standards for being used in explosive area.

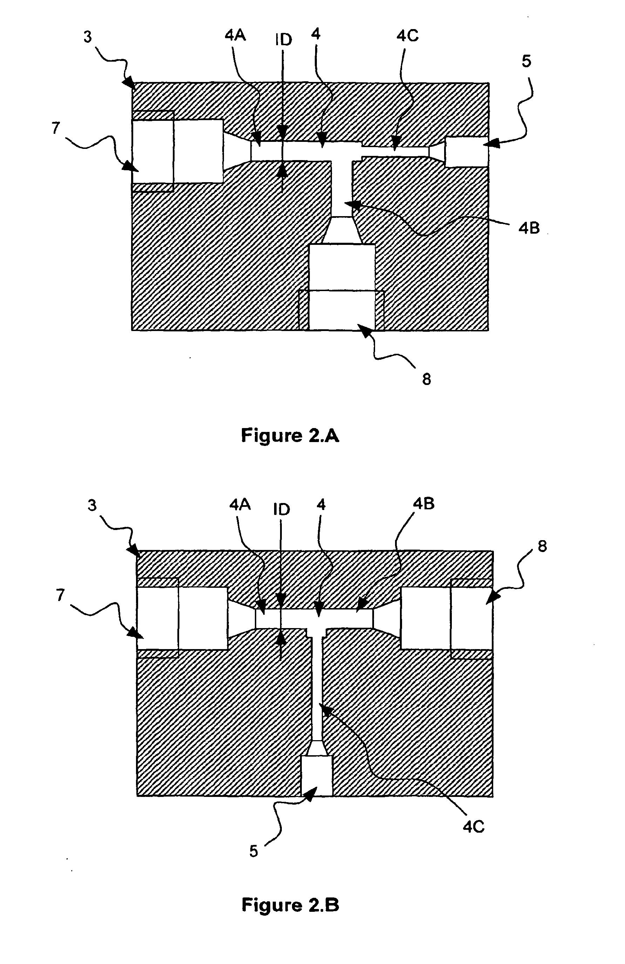

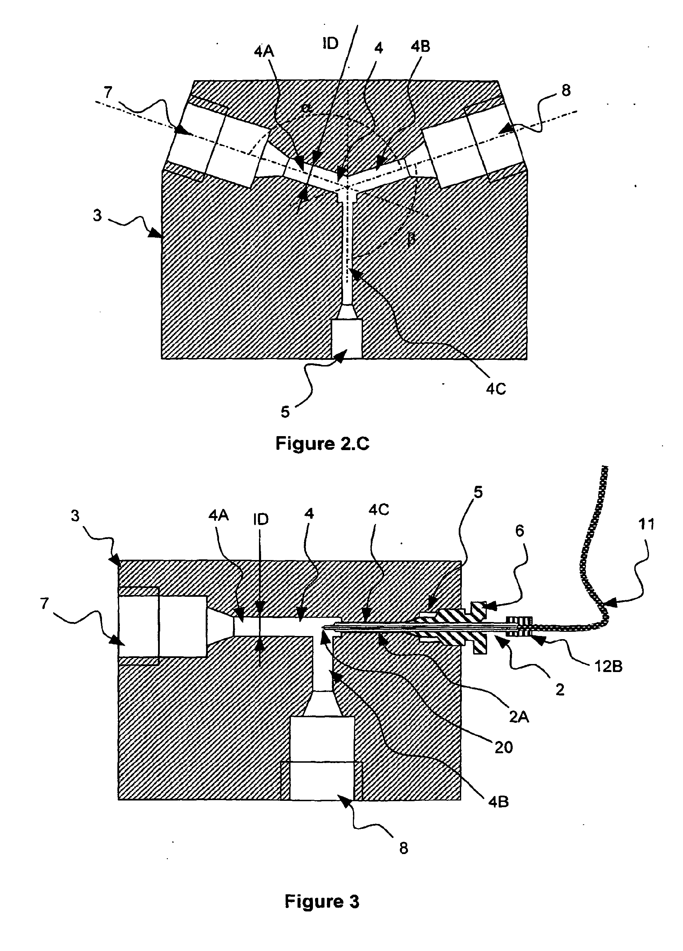

Figures 2.A, 2.B and 2.C schematically illustrate various embodiments of the detector cell of the invention. The detector cell is represented without an optical probe for sake of clarity.
The detector cell has substantially the form of a parallelepiped bloc. Alternatively, the detector cell can be incorporated in a manifold of valves.
The detector cell 3 is arranged to be coupled to the multiphase fluid mixture flowing line (not shown) by means of input connection 7 and output connection 8. The detector cell 3 is also arranged to be coupled to the optical probe (not shown on Figures 2). Obviously, the input connection 7 and output connection 8 can be inverted, the detector being able to perform measurements on a fluid mixture flowing from the input connection 7 towards the output connection 8 and vice versa.

The input connection 7 and output connection 8 of the detector cell are designed for coupling to a flowing line having an internal diameter similar to the typical diameter used in surface sampling and/or PVT (Pressure Volume Temperature) laboratory tubing. Such diameter is typically in the order of one to several millimeters. Advantageously, the coupling between the connection and the flowing line is made by means of high-pressure fittings.
The detector cell further comprises an internal flowing line 4. The internal flowing line is arranged so that each phase of the multiphase fluid mixture, penetrating into the detector cell 3 from the flowing line through the input connection 7, flowing in the internal flowing line 4 are driven-on by capillarity effect. Advantageously, the internal flowing line has an internal diameter ID substantially corresponding to a capillary tube diameter. For example, the internal flowing line 4 has an internal diameter ID ranging from 0,5 mm to 20 mm. However, this range can vary because of the fluid properties. The detector cell further comprises a bore 5 coupled to the internal flowing line 4. The bore enables the positioning of the optical probe in the detector cell.
The internal flowing line 4 and the bore 5 of the detector cell 3 has the shape of a Y. The Y has a first branch 4A, a second branch 4B and a third branch 4C branch, each branch being connected to the two other branches by one extremity. The first branch 4A and second branch 4B are constituted by the internal flowing line 4. The third branch 4C is constituted by the bore 5.

Figure 2.A shows a first embodiment of the detector cell according to which the first branch 4A is perpendicular to the second branch 4B. The third branch 4C is perpendicular to the second branch 4B.

Figure 2.B shows a second embodiment of the detector cell according to which the first branch 4A and the second branch 4B are in-line. The third branch 4C is perpendicular to the second branch 4B.

Figure 2.C shows a third embodiment of the detector cell according to which the first branch 4A forms an angle α respectively to the second branch 4B. The angle α can take a value between 90° (corresponding to the first embodiment) to 180° (corresponding to the second embodiment).
The third branch 4C forms an angle β respectively to the second branch 4B. The angle β can take a value between 0° to 360°-α, with some restrictions due to the space occupied by the internal flowing line.

Figure 3 schematically illustrates the preferred embodiments of the detector cell of the invention comprising an optical probe. The preferred embodiment corresponds to the first embodiment illustrated in Figure 2.A. The first branch 4A is perpendicular to the second branch 4B. The third branch 4C is perpendicular to the second branch 4B or in-line with the first branch 4A.

The optical probe 2 comprises a rod. Advantageously, the rod made of sapphire has a bi-cone shaped tip 20. The sapphire rod is inserted in a sleeve 2A. The sleeve 2A is advantageously made of stainless steel. The optical probe is connected to the optical fiber 11 through an optical connector 12B for handling light coming into the rod and light coming back from the rod after reflection in the tip.
The optical probe 2 is coupled to the bore 5 of the detector cell through a compression fitting 6.

The bore 5 and the compression fitting 6 enable the positioning of the optical probe in the detector cell so that the multiphase fluid mixture flowing in the internal flowing line 4, flows substantially around the tip 20 of the optical probe. The compression fitting also ensures a good sealing between high-pressure fluids flowing into the internal flowing line of the detector cell and the external pressure (for example the atmosphere pressure).
The preferred embodiment enables an efficient auto-cleaning of the tip 20 of the optical probe rod. In particular, the risk for the tip 20 to be subject of dirt deposit (e.g. due to heavy oil phase) during a long time is very limited.
Advantageously, all the elements in contact with fluid (detector cell, optical probe and fittings) are made of materials like stainless steel and sapphire that are acknowledged being compliant with the pressure (higher than 700 bar) and temperature (higher than 150 °C) involved, and chemically compatible with the various components expected in the multiphase fluid mixture.

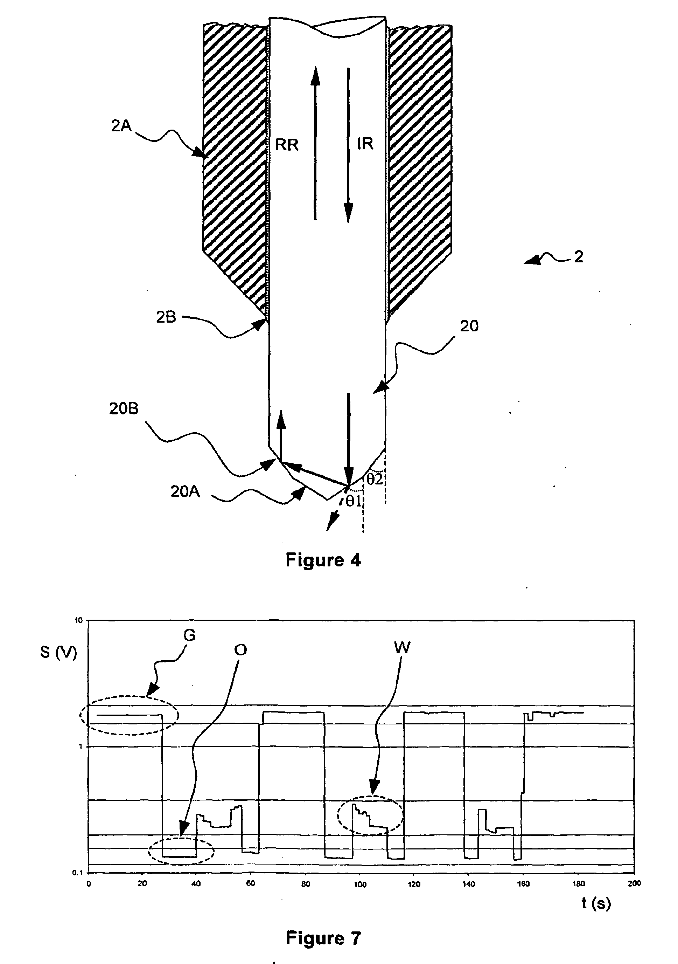

Figure 4 is a longitudinal section view in a part of the optical probe that schematically illustrates the measurement principle of the optical probe. The optical probe is described in US 5,956,132.
The sapphire rod is inserted in a sleeve 2A and maintained together by means of a bounding material 2B. The rod is advantageously made of sapphire and the sleeve 2A of stainless steel. The measurement principle of the probe is based on the differences in refractive index between water, oil and gas phases. Typically, the optical refractive index of multiphase fluid flowing out of a hydrocarbon well is as follows:
- for a gas phase (at the atmospheric pressure), the index ranges between 1.0003 and 1.0020,
- for an oil phase, the index ranges between 1.40 and 1.50,
- for a water phase, the index ranges between 1.33 and 1.35.
   The sapphire refractive index is 1.77, which ensures a sufficient contrast with the various phase refractive indices.

The rod has a bi-cone shaped tip 20. The bi-cone shaped tip 20 comprises a sensitive zone 20A and a total reflection zone 20B. The sensitive zone 20A and the total reflection zone 20B are adjacent and coaxial relatively to the rod longitudinal axis. The sensitive zone 20A forms a first angle θ1 (for example 100°) relatively to the rod longitudinal axis, so that an incident light beam IR is reflected when the probe tip 20 is surrounded with gas and refracted when the probe tip is surrounded with liquid. The total reflection zone 20B forms a second angle θ2 (for example 10°) relatively to the rod longitudinal axis, in order to discriminate between oil and water.
The reflected fraction RR of the incident light beam varies as a function of the refractive index of the phase of the multiphase fluid mixture in which the tip is surrounded.

The optical probe 2 is connected by means of optical fiber 11 to the electronic module 10 comprising the emitting and detection circuits. The detector provides a signal which level is specific to the fluid phase in contact with the optical probe tip.
When the probe is surrounded by gas, typically 36 % of the incident light beam is reflected in the sapphire tip. The reflected light beam is detected as a high level signal by the electronic module 10.
When the probe is surrounded by oil or gasoline, typically more than 99% of the incident light beam is refracted in the surrounding liquid. The reflected light beam is detected as a low level signal (less than 1%).
When the probe is surrounded by water, typically 14 % of the incident light beam is reflected. Thus a discrimination of water relatively to oil and gas is possible (as shown in Figure 7 representing a typical signal evolution versus time).

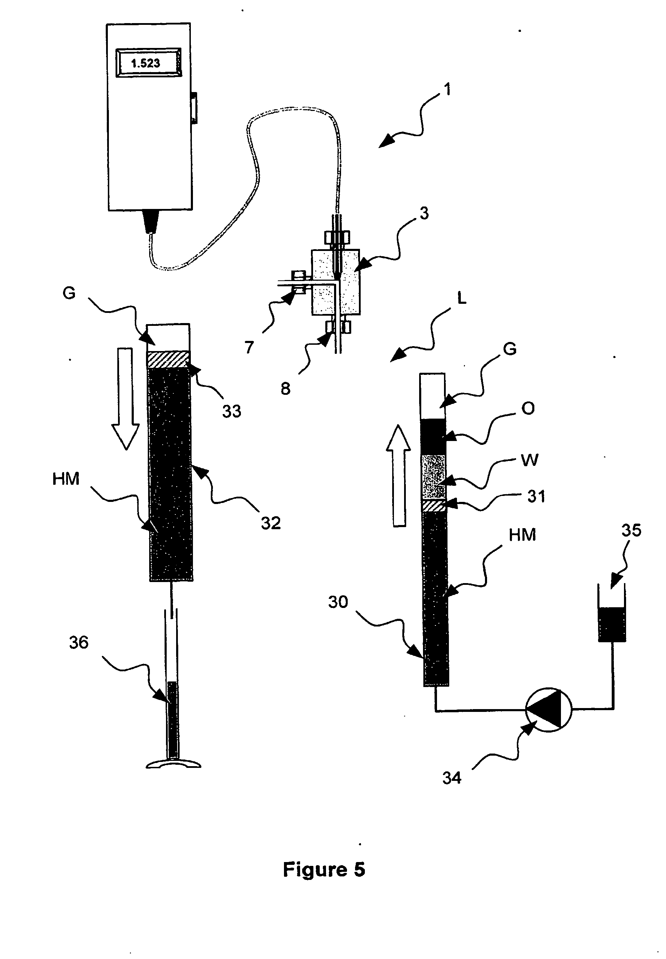

Figure 5 schematically illustrates a first application of the invention.
Identifying phases during sampling operation is a constant concern during oilfield operations. In effect, for economical reasons, it is necessary to take a sample of the desired fluid phase before any future fluid comprehensive laboratory analysis is performed. Further, it is desirable that each sampled phase is not polluted with another. Pollution can easily occur:
- because of mass transfer downstream of a sampling valve due to pressure and temperature change, or
- when the sampling is performed in a multiphase meter that does not enable to collect a pure phase, or
- during fluid transfer from a vessel to another vessel (sampler, shipping cylinder, PVT cell, pycnometer, flash apparatus, etc...).
   For example, when using a down-hole sampling tool, it is necessary to take a specific phase of the fluid mixture or to monitor which phases are flowing into a sampling or shipping bottle.
   In the first application the detector of the invention is used as a stand-alone phase interface sensing detector. Thus, it is possible to sense the interfaces between gas, oil and water for avoiding polluting the transferred sample with another unwanted one. Typically, in this application, the detector is operated in full autonomy (battery operation) and the operator uses visual signal to differentiate between the phase.

A multiphase fluid mixture is transferred from a sampling bottle 30 or a down hole sampler into a shipping bottle 32. The shipping bottle is subsequently shipped to a laboratory for a detailed analysis of the fluid properties. During such a process, it is important to ensure that the fluid, generally the one containing hydrocarbon, transferred into the shipping bottle is in a single phase (gas or liquid) and to avoid transferring water.
Once the multiphase fluid mixture has settle by gravity into a gas G, oil/condensate O and water W layer, the sampling bottle 30 is coupled to the shipping bottle 32 by means of a flow line L. The sampling 30 and the shipping 32 bottles comprise respective pistons 31 and 33. The shipping bottle is initially filled with a hydraulic media HM (e.g. water with glycol). During the transferring process, a transfer pump 34 draw up the hydraulic media HM from a hydraulic media tank 35 to the sampling bottle 30, thereby pushing the piston 31 and transferring a phase (e.g. gas G) to the shipping bottle. The phase (e.g. gas G) flowing into the shipping bottle pushes the piston 33, causing the hydraulic media HM to flow into a test tube 36. Advantageously, the test tube is graduated for volume measurement.

The detector 1, in particular the detector cell 3 is inserted into a flow line L using the high-pressure connections 7 and 8. The detector is used for sensing the interface between gas G and liquid (condensate/oil O or water W) or between oil/condensate O and water W, in order to stop transfer before a second phase enters the shipping bottle 32.
The first application was described by detailing a particular transfer between a sampling and a shipping bottle. However, the detector can be used for any transfer between any vessel (for example PVT laboratories, pycnometer, flash apparatus, etc... ).

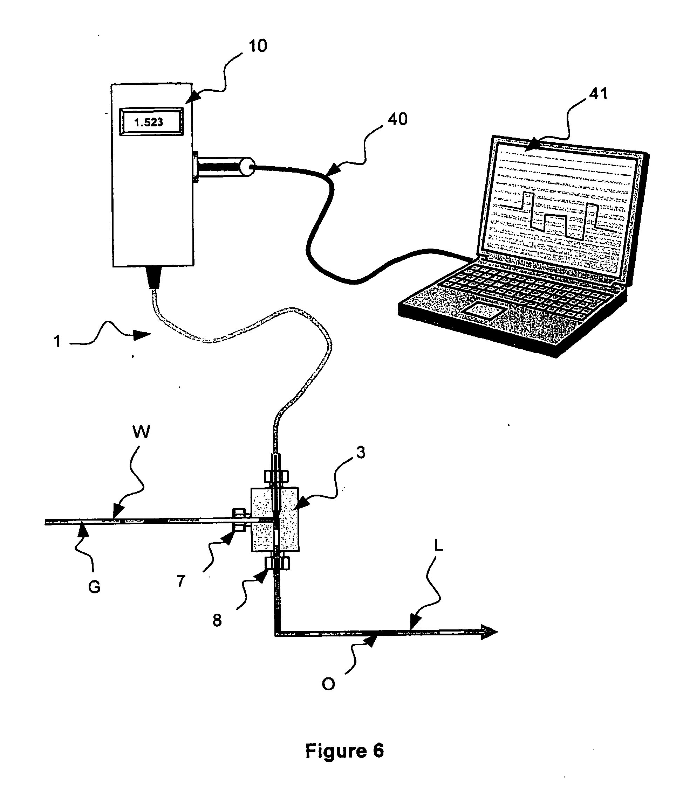

Figure 6 schematically illustrates a second application of the invention.
Measuring the phase ratios during oilfield operations is an important concern. In particular, the accurate measurement of the phase ratios in the main pipe is difficult to perform when one main phase is present in the pipe (e.g. high gas volume fraction, wet gas flow or the opposite).

In this application, the detector 1 is used for determining the different combinations of gas, water, oil/condensate ratios at line conditions in a flow line L, which is derived from a main flow line (not shown). The in-line phase detector is used for the continuous monitoring of the fluid phases flowing through the line, by logging the signal versus time.
The detector 1, in particular the detector cell 3 is inserted into a flow line L using the high-pressure connections 7 and 8. The detector 1, in particular the electronic module 10, is connected to an acquisition device 41 (e.g. a computer) for recording the signal during a flow period.
Due to the detector cell internal flowing line diameter, wettability and capillarity effect, the phases of the multiphase fluid mixture are flowing as plugs and rings, and the fluid is moving at a relatively constant speed (typically a few cm/s). Further, the optical probe material limits any wettability effect. These conditions and properties result in a quasi square signal having three levels corresponding respectively to gas G, water W and oil/condensate O. Figure 7 shows a typical signal S(V) over time t(s) graphic obtained in the second application of the invention illustrated in Figure 6. Further, the signal amplitude ratio enables to calculate the relevant volumetric fraction of each phase.

In the case of a very strong emulsion (typically oil and water), the signal S(V) has an intermediate level between the two possible values. An estimate based on the signal amplitude ratio could give the relevant volumetric fraction of each phase.
In the case of a stable emulsion where the particles (droplets) size is much smaller than the probe tip dimension, the signal S(V) is proportional to the water-oil ratio. The detector could be used for measuring the water-oil ratio.

The in-line phase detector could be used to calibrate, recalibrate or compensate any instruments on line measuring oil/condensate, gas, water or any combination fractions which could be affected by some of the fluid properties (e.g. a dual gamma ray measurement apparatus is affected water salinity changes) or by ageing electronics providing a different response versus time to any measuring or main acquisition device.
Further, it could also be used to compensate automatically any deposits on any probe or window permanently installed online.

In a third application (not illustrated), the detector according to the invention is used to perform flow rate measurements.
The detector may be coupled with an isokinetic sampling device to give in real time the flow rate of each phase flowing in the sampling pipe.
Further two or more detectors may be mounted in series in order to measure a volumetric flow rate, providing that there are more than one phase present in the flow line. The detectors are connected to a computer or any other acquisition device which can perform a cross correlation of the detectors signals and determine the transit time of each phase from one detector to the other. These data are subsequently computed to give a measurement of the volumetric flow rate in the sampling line.

In the description made hereinbefore, particular multiphase fluid mixture related to an effluent flowing from a hydrocarbon well typically comprises three phases has been described. However, it will be evident for one ordinary skilled in the art that the invention is also applicable to other kind of multiphase fluid mixture, for example in the chemical industry or the food industry. Further, examples of internal flowing line dimension were given in the field of oil industry. However, it will be evident for one ordinary skilled in the art that this dimension varies depending on the fluid mixture properties and that this dimension has to be adapted to maintain the capillarity effect in the internal flowing line.
Finally, the term main line use in the present description has a broad meaning, and covers line having a dimension of a few centimeters, for example pipeline, sub-sea line, down-hole line, sampling line, etc...

The drawings and their description hereinbefore illustrate rather than limit the invention. Any reference sign in a claim should not be construed as limiting the claim. The word "comprising" does not exclude the presence of other elements than those listed in a claim. The word "a" or "an" preceding an element does not exclude the presence of a plurality of such elements.

## Claims

1. A detector (1) for distinguishing a phase from a multiphase fluid mixture, the detector comprising:
- an optical probe (2) for optically distinguishing one of the phases from the multiphase fluid mixture, the optical probe haying a tip (20) in contact with the multiphase fluid mixture, wherein the detector is **characterized in** having:
- a detector cell (3) arranged to be coupled to a multiphase fluid mixture flowing line (L) and to the optical probe (2), wherein the detector cell (3) comprises:
- an internal flowing line (4) having an internal diameter so that the phases of the multiphase fluid mixture flowing in the internal flowing line are driven by capillarity effect, and
- a bore (5) coupled to the internal flowing line for positioning the optical probe in the detector cell so that the multiphase fluid mixture flows substantially around the tip (20) of the optical probe.

2. A detector for distinguishing a phase from a multiphase fluid mixture according to claim 1, wherein the internal flowing line has an internal diameter ranging from 0,5 mm to 20 mm.

3. A detector for distinguishing a phase from a multiphase fluid mixture according to any one of the previous claims, wherein the internal flowing line and the bore of the detector cell forms a Y having a first (4A), a second (4B) and a third (4C) branch, the first and second branch (4A, 4B) being formed by the internal flowing line (4) and the third branch (4C) being formed by the bore (5) of the detector cell.

4. A detector for distinguishing a phase from a multiphase fluid mixture according to claim 3, wherein the first (4A) and second (4B) branch are perpendicular.

5. A detector for distinguishing a phase from a multiphase fluid mixture according to claim 3, wherein the first (4A) and second (4B) branch are in-line.

6. A detector for distinguishing a phase from a multiphase fluid mixture according to claim 3 or 4, wherein the third branch (4C) is perpendicular to the second branch (48).

7. A detector for distinguishing a phase from a multiphase fluid mixture according to any one of the previous claims, wherein the optical probe (2) is coupled to the bore (5) of the detector cell through a compression fitting (6).

8. A detector for distinguishing a phase from a multiphase fluid mixture according to any one of the previous claims, wherein the optical probe comprises a sapphire rod having a bi-cone shaped tip (2A), the optical probe (2) being connected to an electronic module (10) though an optical fiber (11).

9. A transferring arrangement, for transferring a single phase from a sampling bottle (30) comprising a multiphase fluid mixture to a shipping bottle (32), the transferring arrangement comprising:
- a flow line (L) coupling the sampling bottle (30) to the shipping bottle (32), and
- a pumping arrangement (31, 33, 34, 35, HM) for transferring a fluid from the sampling bottle (30) to the shipping bottle (32),
- a detector (1) for distinguishing a phase from a multiphase fluid mixture, the detector comprising:
- an optical probe (2) for optically distinguishing one of the phases from the part of the multiphase fluid mixture, the optical probe having a tip (20) in contact with the fluid transferred from the sampling bottle (30) to the shipping bottle (32),
- a detector cell (3) arranged to be coupled to the flow line (L) and to the optical probe (2) and comprising an internal flowing line (4) and a bore (5) coupled to the internal flowing line;
wherein the internal flowing line (4) has an internal diameter so that the phases of the multiphase fluid mixture flowing in the internal flowing line are driven by capillarity effect and
- the bore (5) positioning the optical probe in the detector cell so that the part of the multiphase fluid mixture flows substantially around the tip (20) of the optical probe.

10. A hydrocarbon well testing arrangement, for testing a well that produces a multiphase fluid mixture flowing through a main line, the arrangement comprising:
- a derivation line (L) coupled to the main line for deriving a part of the multiphase fluid mixture,
- a detector (1) for distinguishing a phase from a multiphase fluid mixture, the detector comprising:
- an optical probe (2) for optically distinguishing one of the phases from the part of the multiphase fluid mixture, the optical probe having a tip (20) in contact with the part of the multiphase fluid mixture,
- a detector cell (3) arranged to be coupled to the derivation line and to the optical probe (2) and comprising an internal flowing line (4) and a bore (5) coupled to the internal flowing line,
- the internal flowing line (4) having an internal diameter so that the phases of the part of the multiphase fluid mixture flowing in the internal flowing line are driven by capillarity effect,
- the bore (5) positioning the optical probe in the detector cell so that the part of the multiphase fluid mixture flows substantially around the tip (20) of the optical probe.
- an acquisition device (41) connected to the detector (1).

11. A hydrocarbon well flowrate measurement arrangement, for measuring a flowrate of a phase of a multiphase fluid mixture flowing through a main line of a hydrocarbon well, the arrangement comprising:
- a derivation line coupled to the main line for deriving a part of the multiphase fluid mixture,
- at least two detectors for distinguishing a phase from a multiphase fluid mixture, each detector being connected in series along the derivation line, each detector comprising:
- an optical probe for optically distinguishing one of the phases from the part of the multiphase fluid mixture, the optical probe having a tip in contact with the part of the multiphase fluid mixture,
- a detector cell arranged to be coupled to the derivation line and to the optical probe and comprising an internal flowing line and a bore coupled to the internal flowing line,
- the internal flowing line having an internal diameter so that the phases of the part of the multiphase fluid mixture flowing in the internal flowing line are driven by capillarity effect in the internal flowing line,
- the bore positioning the optical probe in the detector cell so that the part of the multiphase fluid mixture flows substantially around the tip of the optical probe,
- an acquisition device connected to the detectors, the acquisition device :
- receiving from each detector a signal which level is characteristic of the phase flowing around the tip.
- performing a cross correlation of the received signals,
- determining a transit time of each phase from one detector to the other, and
- computing a measurement representative of the flow rate in the main line.

## Patentansprüche

1. Detektor (1) zum Unterscheiden einer Phase eines mehrphasigen Fluidgemischs, wobei der Detektor umfasst:
- einen optischen Sensor (2), um eine der Phasen des mehrphasigen Fluidgemischs optisch zu unterscheiden, wobei der optische Sensor eine Spitze (20) besitzt, die mit dem mehrphasigen Fluidgemisch in Kontakt ist, wobei der Detektor **dadurch gekennzeichnet ist, dass** er umfasst:
- eine Detektorzelle (3), die dazu ausgelegt ist, mit einer Strömungsleitung (L) für das mehrphasige Fluidgemisch und mit dem optischen Sensor (2) gekoppelt zu werden, wobei die Detektorzelle (3) umfasst:
- eine innere Strömungsleitung (4) mit einem Innendurchmesser, derart, dass die Phasen des mehrphasigen Fluidgemischs, das in die innere Strömungsleitung strömt, durch Kapillarwirkung angetrieben werden, und
- eine Bohrung (5), die mit der inneren Strömungsleitung gekoppelt ist, um den optischen Sensor in der Detektorzelle so zu positionieren, dass das mehrphasige Fluidgemisch im Wesentlichen um die Spitze (20) des optischen Sensors strömt.

2. Detektor zum Unterscheiden einer Phase eines mehrphasigen Fluidgemischs nach Anspruch 1, wobei die innere Strömungsleitung einen Innendurchmesser im Bereich von 0,5 mm bis 20 mm besitzt.

3. Detektor zum Unterscheiden einer Phase eines mehrphasigen Fluidgemischs nach einem der vorhergehenden Ansprüche, wobei die innere Strömungsleitung und die Bohrung der Detektorzelle ein Y mit einem ersten Schenkel (4A), einem zweiten Schenkel (4B) und einen dritten Schenkel (4C) bilden, wobei der erste und der zweite Schenkel (4A, 4B) durch die innere Strömungsleitung (4) gebildet sind und der dritte Schenkel (4C) durch die Bohrung (5) der Detektorzelle gebildet ist.

4. Detektor zum Unterscheiden einer Phase eines mehrphasigen Fluidgemischs nach Anspruch 3, wobei der erste Schenkel (4A) und der zweite Schenkel (4B) zueinander senkrecht sind.

5. Detektor zum Unterscheiden einer Phase eines mehrphasigen Fluidgemischs nach Anspruch 3, wobei der erste Schenkel (4A) und der zweite Schenkel (4B) in einer Linie angeordnet sind.

6. Detektor zum Unterscheiden einer Phase eines mehrphasigen Fluidgemischs nach Anspruch 3 oder 4, wobei der dritte Schenkel (4C) zu dem zweiten Schenkel (4B) senkrecht ist.

7. Detektor zum Unterscheiden einer Phase eines mehrphasigen Fluidgemischs nach einem der vorhergehenden Ansprüche, wobei der optische Sensor (2) mit der Bohrung (5) der Detektorzelle über ein Druck-Fitting (6) gekoppelt ist.

8. Detektor zum Unterscheiden einer Phase eines mehrphasigen Fluidgemischs nach einem der vorhergehenden Ansprüche, wobei der optische Sensor einen Saphirstab mit einer doppelkegelförmigen Spitze (2A) umfasst, wobei der optische Sensor (2) über eine Lichtleitfaser (11) mit einem elektronischen Modul (10) verbunden ist.

9. Umladeanordnung, um eine einzelne Phase von einer Probennahmenflasche (30), die ein mehrphasiges Fluidgemisch enthält, in eine Transportflasche (32) umzuladen, wobei die Umladeanordnung umfasst:
- eine Strömungsleitung (L), die die Probennahmenflasche (30) mit der Transportflasche (32) verbindet, und
- eine Pumpanordnung (31, 33, 34, 35, HM), um ein Fluid von der Probennahmenflasche (30) in die Transportflasche (32) umzuladen,
- einen Detektor (1), um eine Phase eines mehrphasigen Fluidgemischs zu unterscheiden, wobei der Detektor umfasst:
- einen optischen Sensor (2), um eine der Phasen von dem Teil des mehrphasigen Fluidgemischs optisch zu unterscheiden, wobei der optische Sensor eine Spitze (20) besitzt, die mit dem Fluid, das von der Probennahmenflasche (30) zu der Transportflasche (32) umgeladen wird, in Kontakt ist,
- eine Detektorzelle (3), die dazu ausgelegt ist, mit der Strömungsleitung (L) und mit dem optischen Sensor (2) gekoppelt zu werden, und die eine innere Strömungsleitung (4) sowie eine mit der inneren Strömungsleitung gekoppelte Bohrung (5) umfasst,
- wobei die innere Strömungsleitung (4) einen Innendurchmesser besitzt, derart, dass die Phasen des mehrphasigen Fluidgemischs, die in der inneren Strömungsleitung strömen, durch Kapillarwirkung angetrieben werden, und
- die Bohrung (5) den optischen Sensor in der Detektorzelle so positioniert, dass der Teil des mehrphasigen Fluidgemischs im Wesentlichen um die Spitze (20) des optischen Sensors strömt.

10. Kohlenwasserstoffbohrloch-Prüfanordnung, um ein Bohrloch zu prüfen, das ein mehrphasiges Fluidgemisch fördert, das durch eine Hauptleitung strömt, wobei die Anordnung umfasst:
- eine Abzweigleitung (L), die mit der Hauptleitung gekoppelt ist, um einen Teil des mehrphasigen Fluidgemischs abzuzweigen,
- einen Detektor (1), um eine Phase eines mehrphasigen Fluidgemischs zu unterscheiden, wobei der Detektor umfasst:
- einen optischen Sensor (2), um eine der Phasen des Teils des mehrphasigen Fluidgemischs optisch zu unterscheiden, wobei der optische Sensor eine Spitze (20) besitzt, die mit dem Teil des mehrphasigen Fluidgemischs in Kontakt ist,
- eine Detektorzelle (3), die dazu ausgelegt ist, mit der Abzweigungsleitung und mit dem optischen Sensor (2) gekoppelt zu werden, und die eine innere Strömungsleitung (4) sowie eine mit der inneren Strömungsleitung gekoppelte Bohrung (5) umfasst,
- wobei die innere Strömungsleitung (4) einen Innendurchmesser besitzt, derart, dass die Phasen des Teils des mehrphasigen Fluidgemischs, der durch die innere Strömungsleitung strömt, durch Kapillarwirkung angetrieben werden, und
- die Bohrung (5) den optischen Sensor in der Detektorzelle so positioniert, dass der Teil des mehrphasigen Fluidgemischs im Wesentlichen um die Spitze (20) des optischen Sensors strömt, und
- eine Erfassungsvorrichtung (41), die mit dem Detektor (1) verbunden ist.

11. Kohlenwasserstoffbohrloch-Durchflussmengenmessanordung, um eine Durchflussmenge einer Phase eines mehrphasigen Fluidgemischs, das durch eine Hauptleitung eines Kohlenwasserstoffbohrlochs strömt, zu messen, wobei die Anordnung umfasst:
- eine Abzweigleitung, die mit der Hauptleitung gekoppelt ist, um einen Teil des mehrphasigen Fluidgemischs abzuzweigen,
- wenigstens zwei Detektoren, um eine Phase eines mehrphasigen Fluidgemischs zu unterscheiden, wobei die beiden Detektoren längs der Abzweigleitung in Reihe verbunden sind und wobei jeder Detektor umfasst:
- einen optischen Sensor, um eine der Phasen des mehrphasigen Fluidgemischs optisch zu unterscheiden, wobei der optische Sensor eine Spitze besitzt, die mit dem Teil des mehrphasigen Fluidgemischs in Kontakt ist, und
- eine Detektorzelle, die dazu ausgelegt ist, mit der Abzweigleitung und mit dem optischen Sensor gekoppelt zu werden, und die eine innere Strömungsleitung sowie eine mit der inneren Strömungsleitung gekoppelte Bohrung aufweist,
- wobei die innere Strömungsleitung einen Innendurchmesser besitzt, so dass die Phasen des Teils des mehrphasigen Fluidgemischs, das in der inneren Strömungsleitung strömt, durch Kapillarwirkung in der inneren Strömungsleitung angetrieben werden, und
- die Bohrung den optischen Sensor in der Detektorzelle so positioniert, dass der Teil des mehrphasigen Fluidgemischs im Wesentlichen um die Spitze des optischen Sensors strömt,
- eine Erfassungsvorrichtung, die mit den Detektoren verbunden ist, wobei die Erfassungsvorrichtung:
- von jedem Detektor ein Signal empfängt, dessen Pegel für die um die Spitze strömende Phase charakteristisch ist,
- eine Kreuzkorrelation der empfangenen Signale ausführt,
- eine Übergangszeit jeder Phase von einem Detektor zum Nächsten bestimmt und
- eine Messung berechnet, die für die Durchflussmenge in der Hauptleitung repräsentativ ist.

## Revendications

1. Détecteur (1) destiné à distinguer une phase d'un mélange fluide à phases multiples, le détecteur comprenant :
- une sonde optique (2) destinée à distinguer optiquement une des phases du mélange fluide à phases multiples, la sonde optique ayant une pointe (20) en contact avec le mélange fluide à phases multiples, dans lequel le détecteur est **caractérisé en ce qu'**il a :
- une cellule de détecteur (3) agencée pour être couplée à une conduite d'écoulement (L) du mélange fluide à phases multiples et à la sonde optique (2),
dans lequel la cellule de détecteur (3) comprend :
- une conduite d'écoulement interne (4) ayant un diamètre interne de sorte que les phases du mélange fluide à phases multiples s'écoulant dans la conduite d'écoulement interne soient entraînées par effet de capillarité, et
- un alésage (5) couplé à la conduite d'écoulement interne, destiné à positionner la sonde optique dans la cellule de détecteur de sorte que le mélange fluide à phases multiples s'écoule sensiblement autour de la pointe (20) de la sonde optique.

2. Détecteur destiné à distinguer une phase d'un mélange fluide à phases multiples selon la revendication 1, dans lequel la conduite d'écoulement interne possède un diamètre interne allant de 0,5 mm à 20 mm.

3. Détecteur destiné à distinguer une phase d'un mélange fluide à phases multiples selon l'une quelconque des revendications précédentes, dans lequel la conduite d'écoulement interne et l'alésage de la cellule de détecteur forment un Y comportant une première (4A), une deuxième (4B) et une troisième (4C) branches, les première et deuxième branches (4A, 4B) étant formées par la conduite d'écoulement interne (4) et la troisième branche (4C) étant formée par l'alésage (5) de la cellule de détecteur.

4. Détecteur destiné à distinguer une phase d'un mélange fluide à phases multiples selon la revendication 3, dans lequel les première (4A) et deuxième (4B) branches sont perpendiculaires.

5. Détecteur destiné à distinguer une phase d'un mélange fluide à phases multiples selon la revendication 3, dans lequel les première (4A) et deuxième (4B) branches sont en ligne.

6. Détecteur destiné à distinguer une phase d'un mélange fluide à phases multiples selon la revendication 3 ou 4, dans lequel la troisième branche (4C) est perpendiculaire à la deuxième branche (4B).

7. Détecteur destiné à distinguer une phase d'un mélange fluide à phases multiples selon l'une quelconque des revendications précédentes, dans lequel la sonde optique (2) est couplée à l'alésage (5) de la cellule de détecteur par l'intermédiaire d'un raccord à compression (6).

8. Détecteur destiné à distinguer une phase d'un mélange fluide à phases multiples selon l'une quelconque des revendications précédentes, dans lequel la sonde optique comprend une tige de saphir comportant une pointe de forme biconique (2A), la sonde optique (2) étant connectée à un module électronique (10) par l'intermédiaire d'une fibre optique (11).

9. Agencement de transfert, destiné à transférer une phase unique d'une bouteille d'échantillonnage (30) comprenant un mélange fluide à phases multiples à une bouteille d'expédition (32), l'agencement de transfert comprenant :
- une conduite d'écoulement (L) couplant la bouteille d'échantillonnage (30) à la bouteille d'expédition (32), et
- un agencement de pompage (31, 33, 34, 35, HM) pour transférer un fluide de la bouteille d'échantillonnage (30) à la bouteille d'expédition (32),
- un détecteur (1) destiné à distinguer une phase d'un mélange fluide à phases multiples, le détecteur comprenant :
- une sonde optique (2) destinée à distinguer optiquement une des phases de la partie du mélange fluide à phases multiples, la sonde optique comportant une pointe (20) en contact avec le fluide transféré de la bouteille d'échantillonnage (30) à la bouteille d'expédition (32),
- une cellule de détecteur (3) agencée pour être couplée à la conduite d'écoulement (L) et à la sonde optique (2) et comprenant une conduite d'écoulement interne (4) et un alésage (5) couplé à la conduite d'écoulement interne ;
dans lequel la conduite d'écoulement interne (4) a un diamètre interne de sorte que les phases du mélange fluide à phases multiples s'écoulant dans la conduite d'écoulement interne soient entraînées par effet capillaire et
- l'alésage (5) positionnant la sonde optique dans la cellule de détecteur de sorte que la partie du mélange fluide à phases multiples s'écoule sensiblement autour de la pointe (20) de la sonde optique.

10. Agencement d'essai de puits d'hydrocarbure, pour réaliser un essai sur un puits qui produit un mélange fluide à phases multiples s'écoulant à travers une conduite principale, l'agencement comprenant :
- une conduite de dérivation (L) couplée à la conduite principale pour dériver une partie du mélange fluide à phases multiples,
- un détecteur (1) destiné à distinguer une phase d'un mélange fluide à phases multiples, le détecteur comprenant :
- une sonde optique (2) destinée à distinguer optiquement une des phases de la partie du mélange fluide à phases multiples, la sonde optique comportant une pointe (20) en contact avec la partie du mélange fluide à phases multiples,
- une cellule de détecteur (3) agencée pour être couplée à la conduite de dérivation et à la sonde optique (2) et comprenant une conduite d'écoulement interne (4) et un alésage (5) couplé à la conduite d'écoulement interne,
- la conduite d'écoulement interne (4) possédant un diamètre interne de sorte que les phases de la partie du mélange fluide à phases multiples s'écoulant dans la conduite d'écoulement interne soient entraînées par effet de capillarité,
- l'alésage (5) positionnant la sonde optique dans la cellule de détecteur de sorte que la partie du mélange fluide à phases multiples s'écoule sensiblement autour de la pointe (20) de la sonde optique,
- un dispositif d'acquisition (41) connecté au détecteur (1).

11. Agencement de mesure de débit de puits d'hydrocarbure, pour mesurer un débit d'une phase d'un mélange fluide à phases multiples s'écoulant à travers une conduite principale d'un puits d'hydrocarbure, l'agencement comprenant :
- une conduite de dérivation couplée à la conduite principale pour dériver une partie du mélange fluide à phases multiples,
- au moins deux détecteurs destinés à distinguer une phase d'un mélange fluide à phases multiples, chaque détecteur étant connecté en série le long de la conduite de dérivation, chaque détecteur comprenant :
- une sonde optique destinée à distinguer optiquement une des phases de la partie du mélange fluide à phases multiples, la sonde optique comportant une pointe en contact avec la partie du mélange fluide à phases multiples,
- une cellule de détecteur agencée pour être couplée à la conduite de dérivation et à la sonde optique et comprenant une conduite d'écoulement interne et un alésage couplé à la conduite d'écoulement interne,
- la conduite d'écoulement interne ayant un diamètre interne de sorte que les phases de la partie du mélange fluide à phases multiples s'écoulant dans la conduite d'écoulement interne soient entraînées par effet de capillarité dans la conduite d'écoulement interne,
- l'alésage positionnant la sonde optique dans la cellule de détecteur de sorte que la partie du mélange fluide à phases multiples s'écoule sensiblement autour de la pointe de la sonde optique,
- un dispositif d'acquisition connecté aux détecteurs, le dispositif d'acquisition :
- recevant de chaque détecteur un signal dont le niveau est caractéristique de la phase s'écoulant autour de la pointe,
- réalisant une corrélation croisée des signaux reçus,
- déterminant un temps de transit de chaque phase d'un détecteur à l'autre, et
- calculant une mesure représentative du débit dans la conduite principale.
